# EUROPÄISCHE PATENTSCHRIFT

(11) **EP 1 940 359 B1**
(45) Veröffentlichungstag und Bekanntmachung des Hinweises auf die Patenterteilung: **19.07.2017**
(21) Anmeldenummer: 06806988.9
(22) Anmeldetag: 05.10.2006
(51) Int. Cl.: A61K 9/16, A61K 9/20, C07C 57/30

(54) **VERFAHREN ZUR HERSTELLUNG VON DIREKTTABLETTIERBAREN IBUPROFEN-FORMULIERUNGEN**
METHOD FOR THE PRODUCTION OF DIRECTLY COMPRESSIBLE IBUPROFEN FORMULATIONS
PROCEDE POUR PRODUIRE DES FORMULATIONS D'IBUPROFENE POUVANT ETRE DIRECTEMENT MISES SOUS FORME DE COMPRIMES

(30) Priorität: 11.10.2005 DE 102005049001
(43) Veröffentlichungstag der Anmeldung: 09.07.2008
(73) Patentinhaber: BASF SE, 67056 Ludwigshafen am Rhein (DE)
(72) Erfinder: MEYER-BÖHM, Kathrin, 90537 Feucht (DE); KOLTER, Karl, 67117 Limburgerhof (DE); QUADIR, Anisul, Hackettstown, NJ 07840 (US)
(74) Vertreter: BASF IP Association
(86) Internationale Anmeldenummer: PCT/EP2006/067058
(87) Internationale Veröffentlichungsnummer: WO 2007/042445

(56) Entgegenhaltungen:
- WO-A-92/08686
- WO-A-94/10993
- JP-A- 62 294 637
- US-A- 5 191 114
- US-B1- 6 951 657

## Beschreibung

Die vorliegende Erfindung betrifft ein verbessertes Verfahren zur Herstellung von direkttablettierbaren Ibuprofen-Formulierungen durch Mischen des Ibuprofens mit einem hochdispersen Hilfsstoff sowie die entsprechend erhaltenen Formulierungen.

Bei der Herstellung von Tabletten ist die Direkttablettierung ist ein häufig angewandtes Verfahren, da es einfacher und kostengünstiger ist. Daher gewinnt die Direkttablettierung mehr und mehr an Bedeutung.

Ibuprofen wird bis zu 800mg/Tablette verabreicht. Da man eine problemlose Schluckbarkeit gewährleisten möchte, ist der Gehalt an Ibuprofen in Ibuprofentabletten üblicherweise sehr hoch. Dieser hohe Ibuprofengehalt in einer Ibuprofenformulierung bedingt, dass die Eigenschaften z.B. der Tablettiermischung nahezu ausschließlich durch die Eigenschaften des Ibuprofens bestimmt werden. Daher verursacht der geringe Schmelzpunkt des Ibuprofens von nur 75°C bei der Verarbeitung einer Formulierung gravierende Schwierigkeiten wie unter anderem ein Kleben an den Presswerkzeugen und niedrige Tablettiergeschwindigkeiten. Neben der Klebeneigung bedingt der hohe Ibuprofen anteil häufig auch eine schlechte Fließfähigkeit, da die Ibuprofen-Kristalle meist in einer Größe im Bereich von 50µm vorliegen.

Das unerwünschte Phänomen des Klebens an den Presswerkzeugen verstärkt sich mit zunehmender Dauer eines Produktionszykluses. Andererseits wird ein Hersteller aus Kostengründen immer versuchen, einen Produktionszyklus über möglichst viele Stunden laufen zu lassen, ohne beispielsweise Stempelwerkzeuge zu polieren oder auszubauen.

Vor allem auch bei niedrigeren Pressdrücken bereitet die Verarbeitung von ibuprofenhaltigen Mischungen Probleme. Andererseits wird ein pharmazeutischer Hersteller immer anstreben bei möglichst niedrigen Pressdrücken zu arbeiten, um die hochpreisigen Presswerkzeuge vor Abnutzung zu schonen.

Allgemein bereitet Ibuprofen aufgrund seiner durch den relativ niedrigen Schmelzpunkt verursachten Neigung zum Kleben Probleme bei der Verarbeitung. Bei der Herstellung von ibuprofenhaltigen Trockenmischungen kann es vor allem bei hohem Energieeintrag und Erwärmung der Rührer schon beim Mischprozess zum Verkleben der Mischwerkzeuge kommen.

Aus der US 5,191,114 ist ein Verfahren zur Herstellung von Ibuprofen-Pulvern für die Direkttablettierung bekannt, bei dem durch trockenes Vermischen von Ibuprofen mit amorphem Silicagel Pulver mit verbesserter Fließfähigkeit erhalten werden sollen. Wie dem Fachmann bekannt ist, ist die Verbesserung der Fliessfähigkeit auf diese Weise schon nach kurzer Mischzeit zu erzielen. Die Tablettierbarkeit wird auf diese Weise aber praktisch nicht verbessert.

In der EP-A 172 014 ist ebenfalls die Herstellung von ibuprofenhaltigen Formulierungen beschrieben, wobei das Ibuprofen mit Natrium-Croscarmellose als Sprengmittel und geringen Mengen an kolloidaler Kieselsäure bei kurzen Mischzeiten im Bereich weniger Minuten vermischt und anschließend walzenkompaktiert wird. Auch bei diesen Formulierungen wird keine ausreichende Verbesserung der Tablettierbarkeit erzielt.

In der WO 2005/037192 ist ebenfalls die Herstellung von ibuprofenhaltigen Granulaten durch trockenes Vermischen des Wirkstoffs mit pharmazeutischen Hilfsmitteln und anschließender Walzenkompaktierung beschrieben.

Aufgabe der vorliegenden Erfindung war es, verbesserte Ibuprofen-Formulierungen sowie ein verbessertes Verfahren zur Herstellung von direktverpressbarem Ibuprofen zu finden.

Demgemäß wurde ein Verfahren zur Herstellung von direktverpressbaren Ibuprofen-Formulierungen durch Vermischen von Ibuprofen mit einem feindispersen Hilfsstoff, welches dadurch gekennzeichnet ist, dass man eine Mischung aus 50 bis 99 Gew.-% eines kristallinen Ibuprofens mit 1 bis 15 Gew.-% eines hochdispersen Hilfsstoffs mit einer Oberfläche von 100 bis 300 m²/g und 0 bis 40 Gew.-% weiterer Hilfsstoffe, wobei die Gesamtmenge an Mischungskomponenten 100 Gew.-% beträgt, bis die Oberfläche der Ibuprofen-Kristalle zu mindestens 50 % mit dem feindispersen Hilfsstoff belegt ist.

Weiterhin betrifft die Erfindung direkttablettierbare Ibuprofen-Formulierungen, die nach dem erfindungsgemäßen Verfahren erhalten werden.

Erfindungsgemäß kann das Ibuprofen in Form der freien Säure oder als Salz eingesetzt werden, wobei als Salze Alkali- oder Erdalkalisalze oder Salze mit einem basischen Amin oder in Form von Aminosäuresalzen, beispielsweise Lysinat-Salze, in Betracht kommen. Bevorzugte Salze sind die Natrium- und Kalium-Salze, insbesondere Natriumibuprofenat. Das Ibuprofen wird erfindungsgemäß in Form kristalliner Partikel eingesetzt. Die mittlere Teilchengröße der Ibuprofen-Partikel beträgt vorzugsweise 20 bis 200 µm, besonders bevorzugt 25 bis 110 µm.

Das erfindungsgemäße Verfahren wird vorzugsweise so durchgeführt, dass mindestens 60 % besonders bevorzugt mindestens 70 % der Oberfläche der Ibuprofenkristalle mit dem hochdispersen Hilfsstoff belegt sind.

Der hochdisperse Hilfsstoff kann eine spezifische Oberfläche von 100 bis 300 m²/g (gemessen nach der BET-Methode), vorzugsweise mindestens 150m²/g aufweisen, besonders bevorzugt mindestens 200 m²/g, ganz besonders bevorzugt von mindestens 250 m²/g aufweisen. Die mittlere Teilchengröße der Primärpartikel des Hilfsstoffs kann 2 bis 200 nm, bevorzugt 5 bis 100 besonders bevorzugt 5 bis 50 nm betragen.

Als hochdisperse Hilfsstoffe kommen insbesondere Metalloxide in Betracht, bevorzugt Oxide ausgewählt aus der Gruppe der Oxide des Aluminiums, Siliciums, Zinks und des Titans. Besonders bevorzugt sind Oxide des Siliciums, wobei neben hochdisperser Kieselsäure auch hydrophobisierte Kieselsäuren eingesetzt werden können. Hydrophobisierte Kieselsäuren können beispielsweise durch Umsetzung der Silanolgruppen mit Dichlordimethylsilan, Octylsilan oder Hexamethyldisilazan erhalten werden. Ganz besonders bevorzugt wird hochdisperse Kieselsäure eingesetzt, insbesondere mit mittleren Partikelgrößen der Primärpartikel von 5 bis 50 nm.

Die erfindungsgemäßen Ibuprofenformulierungen können weitere übliche Hilfsstoffe wie Füllstoffe, Bindemittel, Sprengmittel und Schmiermittel oder Mischungen davon, enthalten.

Als Füllstoffe können mikrokristalline Cellulose, Cellulose, Calciumhydrogenphosphat, Mannit, Sorbit, Xylit oder Lactose eingesetzt werden, bevorzugt mikrokristalline Cellulose.

Als Bindemittel können Polyvinylpyrrolidon, Vinylpyrrolidon-Vinylacetat-Copolymere, Hydroxypropylmethylcellulose, Hydroxypropylcellulose, Hydroxyethylcellulose oder Methylcellulose eingesetzt werden. Mikrokristalline Cellulose kann auch als Bindemittel eingesetzt werden.

Als Sprengmittel eignen sich quervernetzte Natriumcarboxymethylstärke, quervernetzte Natriumcarboxymethylcellulose (Croscarmellose-Natrium) oder quervernetztes Polyvinylpyrrolidon.

Als Schmiermittel können Stearinsäure, Magnesiumstearat, Natriumstearylfumarat, Leucin, Natriumbenzoat oder Poloxamere eingesetzt werden.

Die erfindungsgemäßen Ibuprofenformulierungen enthalten bevorzugt
a) 50 - 94 Gew.-% Ibuprofen oder Ibuprofensalz,
b) 1 - 15 Gew.-% hochdisperse Kieselsäure mit einer Oberfläche von größer 150 m²/g,
c) 5 - 20 Gew.-% mikrokristalline Cellulose
d) 0 - 10 Gew.-% quervernetztes Polyvinylpyrrolidon, quervernetzte Natriumcarboxymethylcellulose oder quervernetzte Natriumcarboxymethylstärke
e) 0 - 10 Gew.-% Hydroxyalkylcellulose, und
f) 0 - 5 Gew.-% eines üblichen Schmiermittels.

Ganz besonders bevorzugt sind Ibuprofen-Formulierungen enthaltend
a) 50 - 93,5 Gew.-% Ibuprofen oder Ibuprofensalz,
b) 1,5 - 10 Gew.-% hochdisperse Kieselsäure mit einer Oberfläche von größer 150 m²/g,
c) 5 - 15 Gew.-% mikrokristalline Cellulose
d) 0 - 10 Gew.-% quervernetztes Polyvinylpyrrolidon, quervernetzte Natriumcarboxymethylcellulose oder quervernetzte Natriumcarboxymethylstärke
e) 0 - 10 Gew.-% Hydroxyalkylcellulose
f) 0 - 5 Gew.-% eines üblichen Schmiermittels

Das erfindungsgemäße Verfahren zur Herstellung von Ibuprofenformulierungen wird so durchgeführt, dass zunächst Ibuprofen oder Ibuprofensalz mit dem hochdispersen Hilfsstoff und gegebenenfalls weiteren Hilfsstoffen mindestens 30 min, gemischt wird und danach gewünschtenfalls weitere Zusatzstoffe untergemischt werden. Gemäß einer bevorzugten Ausführungsform wird zunächst das Ibuprofen mit dem hochdispersen Hilfsstoff gemischt.

Das Verfahren wir vorzugsweise so durchgeführt, dass bei den jeweiligen Mischprozessen die Temperatur des Ibuprofen-Mischguts 50°C, bevorzugt 40 °C, besonders bevorzugt 35 °C, nicht überschreitet.

Die Mischprozesse können in jeder herkömmlichen Mischvorrichtung erfolgen. Bevorzugt werden niedrig scherende Mischwerkzeuge eingesetzt. Besonders bevorzugt werden Freifallmischer eingesetzt, bei denen die Vermischung durch bewegen des Mischguts in horizontaler oder vertikaler Richtung erfolgt. Insbesondere eignen sich Turbulamischer, Containermischer oder V-Mischer. Die Mischer können auch Mischhilfen in fest montierter oder in beweglicher Form enthalten, die zu einem intensivierten Mischprozess führen. Fest montierte Mischhilfen sind Einbauten verschiedenster Geometrie; unter beweglichen Mischhilfen werden Gegenstände wie beispielsweise Kugeln oder Zylinder oder ähnliche Formkörper verstanden, die ins Mischgut gegeben werden, mitgemischt werden und so den Mischprozess intensivieren.

Die Mischzeit beträgt mindestens 0,5 h. Sie kann von 0,5 h bis zu 8 h betragen, vorzugsweise 1 h bis 5 h, besonders bevorzugt 1,25 h bis 3 h. Welche Mischzeit im Einzelfall gewählt wird, hängt neben dem Mischertyp und dessen Energieeintrag auch von der Menge an eingesetztem hochdispersen Hilfsstoff ab.

Die so erhaltenen Partikel weisen wie beschrieben eine Belegungsdichte an hochdispersem Hilsstoff von mindestens 50 % der Oberfläche der Ibuprofen-Kristalle auf. Die Beurteilung der Oberflächenbelegung erfolgt optisch, vorzugsweise mit Hilfe eines Bildanalysesystems. Dieses Verfahren beruht auf der Auswertung von REM-Aufnahmen (REM: Rasterelektronenmikroskopie).

Gemäß einer bevorzugten Ausführungsform der Erfindung werden die so erhaltenen mit hochdispersem Hilfsstoff belegten Ibuprofen-Kristalle durch Walzenkompaktierung zu Agglomeraten verdichtet, wobei die verdichteten Agglomerate im folgenden auch als Kompaktate bezeichnet werden. Anschließend werden die Kompaktate durch ein Sieb mit einer Maschenweite von 0,8 bis 2,5 mm gedrückt, so dass ein Granulat entsteht.

Die erfindungsgemäßen Ibuprofenformulierungen liegen in der agglomerierten Form mit einer mittleren Teilchengröße von 150 µm bis 1500 µm, bevorzugt 200 µm bis 1200 µm, besonders bevorzugt 250 µm bis 1000 µm, vor. Dabei beträgt der Feinanteil, also der Anteil an Teilchen mit einer Teilchengröße unter 85 µm, vorzugsweise kleiner 10 Gew.-%.

Gemäß einer weiteren bevorzugten Ausführungsform der Erfindung wird dem Granulat in einem zweiten Mischschritt ein weiterer Anteil von mindestens 0,5 Gew.-% und bis zu 5 Gew.-%, bezogen auf die Menge an Kompaktat, an hochdispersem Hilfsstoff zugemischt. Die Mischzeit kann zwischen 0,3 h und 2 h betragen, üblicherweise liegt sie im Bereich von 1 h. Dieser zweite Mischschritt erfolgt vorzugsweise ebenfalls in einem Freifallmischer.

Pharmazeutische Darreichungsformen enthaltend eine erfindungsgemäße Ibuprofenformulierung können auf herkömmliche Weise durch Verpressung der erfindungsgemäß erhaltenen Ibuprofen-Formulierungen, gegebenenfalls unter Zumischung weiterer Hilfsstoffe, erfolgen. Als solche weiteren Hilfsstoffe kommen übliche Füll- und Bindemittel, Sprengmittel, Schmiermittel, oberflächenaktive Substanzen, Fließregulierungsmittel und Geschmackskorrigenzien in Betracht.

Der Ibuprofengehalt der so hergestellten Tablette soll mindestens 60 Gew.-%, bezogen auf das Tablettengewicht, betragen. Für Tabletten mit höheren Wirkstoffdosierungen kann sich auch ein höherer Ibuprofengehalt empfehlen, um das Tablettengewicht niedrig zu halten.

Die Wirkstofffreisetzung aus den Tabletten beträgt mindestens 80 % nach 10 min, bevorzugt mindestens 80 % nach 5 min, gemessen nach dem Paddle-Modell bei 37 °C in Phosphatpuffer mit pH 7,2 gemäß USP 28 oder gemäß der Ph. Eur. (Europäische Pharmakopoe). Die Zerfallszeit der Tabletten in wässrigem Medium liegt bei kleiner 2 min.

Gewünschtenfalls können die Tabletten auch mit einem Filmüberzug versehen werden.

Die erfindungsgemäßen Ibuprofenformulierungen weisen ein deutlich verbessertes Verarbeitungsverhalten bei der Direkttablettierung auf.

### Beispiele

### Beispiel 1

3,5 Gew.-% hochdisperse Kieselsäure ¹⁾, 6,77 Gew.-% mikrokristalline Cellulose und 3 Gew.-% Croscarmellose-Natrium wurden durch ein 800 µm-Sieb gesiebt und in einen Turbula-Mischbehälter (T10B, 780mm x 955mm x 845mm) vorgelegt. 86,72 Gew.-% Ibuprofen wurden ebenfalls durch ein 800 µm Sieb gesiebt und in den Mischer gegeben (Gesamtmasse: 5,0 kg). Anschließend wurde die Mischung 180 min gemischt. Die Belegung der Oberfläche betrug 85 %. Die so erhaltene Vormischung wurde in einem Kompaktor (Minipactor 1114, Gerteis) mit einer Kompaktierkraft von 6 kN/cm für 3 h kompaktiert.
49 Gew.-% des kompaktierten Materials wurden in einem Turbula-Mischbehälter (T10B, 780mm x 955mm x 845mm) vorgelegt. 2 Gew.-% Kieselsäure wurden zugegeben und anschließend weitere 49 Gew.-% des Kompaktats hinzugefügt (Gesamtmasse: 5,0 kg = 100 Gew.-%). Danach wurde für 60 Minuten nachgemischt. Die Belegung der Oberfläche der Agglomerate mit hochdisperser Substanz betrug > 90 %.

¹⁾Aerosil 200, Fa. Degussa: spezifische Oberfläche (BET) 200 m²/g

### Beispiele 2 bis 4

Eine Mischung aus 1 Gew.- % Aerosil 200 und 99 Gew.-% Ibuprofen 50 (mittlere Teilchengröße 50 µm) wurde im Turbulamischer 3 Minuten gemischt und dann durch ein 1,00mm Sieb gesiebt.

Anschließend wurden diese Mischung mit unterschiedlichen Mischzeiten im Turbulamischer gemischt:
Beispiel 2) 10 Minuten
Beispiel 3) 30 Minuten
Beispiel 4) 180 Minuten

Die so erhaltenen Vormischungen wurden in einem Turbulamischer für einen Zeitraum von 10 Minuten mit den im folgenden genannten Mengen an Hilfsstoffen zu einer Pressmischung verarbeitet:

| Zusammensetzung der Pressmischung (Ibuprofen-Gehalt: 75 Gew.-%) | Gehalt [Gew.-%] |
|---|---|
| Vormischung | 75,76 |
| mikrokristalline Cellulose | 20,24 |
| Croscarmellose-Natrium | 3,0 |
| Aerosil 200 | 0,5 |
| Mg-Stearat | 0,5 |

Diese Pressmischungen wurden auf einer Rundläuferpresse Korsch PH 106 bei einem Pressdruck von 15kN verpresst (30 Upm, Stempel: 9 mm, flach gewölbt, ohne Gravur).

| | |
|---|---|
| Pressmischung mit Vormischung gemäß Bsp. 2; Oberflächenbelegung 20 % | Kleben nach 60 Minuten Presszeit⁺⁾ |
| Pressmischung mit Vormischung gemäß Bsp. 3; Oberflächenbelegung 54 % | Kleben nach 4 Stunden Presszeit |
| Pressmischung mit Vormischung gemäß Bsp. 4; Oberflächenbelegung > 85 % | Kein Kleben nach einer Presszeit von 8 h |

| | |
|---|---|
| ⁺⁾Laufzeit der Tablettenpresse | |

## Patentansprüche

1. Direkttablettierbare Ibuprofenformulierung, enthaltend
a) 50 - 99 Gew.-% kristallines Ibuprofen,
b) 1 - 15 Gew.-% eines hochdispersen Hilfsstoffs mit einer Oberfläche von mindestens 100 m²/g, und
c) 0 - 40 Gew.-% weiterer Hilfsstoffe,
mit der Maßgabe, dass die Gesamtmenge der Komponenten a) bis c) 100 Gew.-% entspricht, wobei als hochdisperser Hilfsstoff Kieselsäure enthalten ist, und wobei mindestens 70% der Oberfläche der Ibuprofenkristalle mit dem hochdispersen Hilfsstoff belegt sind.

2. Ibuprofenformulierung nach Anspruch 1, wobei das Ibuprofen in Form der freien Säure oder als Alkali- oder Erdalkalisalz oder als Salz mit einem basischen Amin oder einer Aminosäure vorliegt.

3. Ibuprofenformulierung nach Anspruch 1 oder 2, wobei die Ibuprofen-Kristalle eine mittlere Korngröße von 20 bis 200 µm aufweisen.

4. Ibuprofenformulierung nach einem der Ansprüche 1 bis 3, **dadurch gekennzeichnet, dass** der hochdisperse Hilfsstoff eine Oberfläche von mindestens 150 m²/g aufweist.

5. Ibuprofenformulierung nach einem der Ansprüche 1 bis 4, **dadurch gekennzeichnet, dass** der hochdisperse Hilfsstoff eine Oberfläche von mindestens 200 m²/g aufweist.

6. Ibuprofenformulierung nach einem der Ansprüche 1 bis 5, **dadurch gekennzeichnet, dass** der hochdisperse Hilfsstoff eine Oberfläche von mindestens 250 m²/g aufweist.

7. Ibuprofenformulierung nach einem der Ansprüche 1 bis 6, enthaltend als hochdispersen Hilfsstoff hydrophobisierte Kieselsäure.

8. Ibuprofenformulierung nach einem der Ansprüche 1 bis 7, enthaltend
a) 50 - 94 Gew.-% Ibuprofen,
b) 1 - 15 Gew.-% hochdisperse Kieselsäure mit einer Oberfläche von mindestens 100 m²/g,
c) 5 - 20 Gew.-% mikrokristalline Cellulose,
d) 0 - 10 Gew.-% quervernetztes Polyvinylpyrrolidon, quervernetzte Natriumcarboxymethylcellulose oder quervernetzte Natriumcarboxymethylstärke,
e) 0 - 10 Gew.-% Hydroxyalkylcellulose, und
f) 0 - 5 Gew.-% eines üblichen Schmiermittels.

9. Ibuprofenformulierung nach einem der Ansprüche 1 bis 8, enthaltend
a) 50 - 93,5 Gew.-% Ibuprofen,
b) 1,5 - 10 Gew.-% hochdisperse Kieselsäure mit einer Oberfläche von größer 150 m²/g,
c) 5 - 15 Gew.-% mikrokristalline Cellulose,
d) 0 - 10 Gew.-% quervernetztes Polyvinylpyrrolidon, quervernetzte Natriumcarboxymethylcellulose oder quervernetzte Natriumcarboxymethylstärke
e) 0 - 10 Gew.-% Hydroxyalkylcellulose, und
f) 0 - 5 Gew.-% eines üblichen Schmiermittels.

10. Ibuprofenformulierung nach einem der Ansprüche 1 bis 9, **dadurch gekennzeichnet, dass** als weitere übliche Hilfsstoffe Füllstoffe, Bindemittel, Sprengmittel und Schmiermittel oder Mischungen davon eingesetzt werden.

11. Ibuprofenformulierung nach einem der Ansprüche 1 bis 10, **dadurch gekennzeichnet, dass** als Füllstoffe mikrokristalline Cellulose, Cellulose, Calciumhydrogenphosphat, Mannit, Sorbit, Xylit oder Lactose eingesetzt werden.

12. Ibuprofenformulierung nach einem der Ansprüche 1 bis 11, **dadurch gekennzeichnet, dass** als Bindemittel Polyvinylpyrrolidon, Vinylpyrrolidon-Vinylacetat-Copolymere, Hydroxypropylmethylcellulose, Hydroxypropylcellulose, Hydroxyethylcellulose oder Methylcellulose eingesetzt werden.

13. Ibuprofenformulierung nach einem der Ansprüche 1 bis 12, **dadurch gekennzeichnet, dass** als Sprengmittel quervernetzte Natriumcarboxymethylstärke, quervernetzte Natrimcarboxymethylcellulose oder quervernetztes Polyvinylpyrrolidon eingesetzt werden.

14. Ibuprofenformulierung nach einem der Ansprüche 1 bis 13, **dadurch gekennzeichnet, dass** als Schmiermittel Stearinsäure, Magnesiumstearat, Natriumstearylfumarat, Leucin, Natriumbenzoat oder Poloxamere eingesetzt werden.

15. Ibuprofenformulierung nach einem der Ansprüche 1 bis 14, wobei die mit dem hochdispersen Hilfsstoff belegten Ibuprofen-Kristalle in agglomerierter Form mit einer mittleren Teilchengröße der Agglomerate zwischen 150 und 1500 µm vorliegen.

16. Ibuprofenformulierung nach einem der Ansprüche 1 bis 15, wobei die Agglomerate mit einer mittleren Teilchengröße zwischen 200 und 1200 µm vorliegen.

17. Ibuprofenformulierung nach einem der Ansprüche 1 bis 16, wobei die Agglomerate mit einer mittleren Teilchengröße zwischen 250 und 1000 µm vorliegen.

18. Ibuprofenformulierung nach einem der Ansprüche 1 bis 17, wobei die Agglomerate mit einer mittleren Teilchengröße zwischen 250 und 1000 µm vorliegen und der Anteil an Agglomeraten mit Teilchengrößen < 85 µm weniger als 10 Gew.-% beträgt.

19. Ibuprofenformulierung nach einem der Ansprüche 1 bis 18, wobei die Agglomerate mit einer mittleren Teilchengröße zwischen 250 und 1000µm vorliegt und der Anteil an Agglomeraten < 85 µm weniger als 6 Gew.-% beträgt.

20. Ibuprofenformulierung nach einem der Ansprüche 1 bis 19, wobei die mit dem hochdispersen Hilfsstoff belegten Ibuprofen-Kristalle in agglomerierter Form vorliegen und die Oberfläche der Agglomerate zu mindestens 70% mit dem hochdispersen Hilfsstoff belegt ist.

21. Verfahren zur Herstellung von Ibuprofenformulierungen gemäß einem der Ansprüche 1 bis 20, durch Vermischen von Ibuprofen mit einem hochdispersen Hilfsstoff, **dadurch gekennzeichnet, dass** Ibuprofen mit einem hochdispersen Hilfsstoff, der eine Oberfläche von mindestens 100 m²/g aufweist, vermischt wird, bis mindestens 70 % der Oberfläche der Ibuprofen-Partikel mit dem hochdispersen Hilfsstoff belegt sind.

22. Verfahren nach Anspruch 21, **dadurch gekennzeichnet, dass** Ibuprofen und hochdisperser Hilfsstoff mit einer Mischzeit von 1 h bis 5 h gemischt werden.

23. Verfahren nach Anspruch 21 oder 22, **dadurch gekennzeichnet, dass** zunächst Ibuprofen oder Ibuprofensalz mit hochdisperser Kieselsäure 1 h bis 5 h gemischt wird und dann die restlichen Zusatzstoffe untergemischt werden.

24. Verfahren zur Herstellung von Ibuprofenformulierungen einem der Ansprüche 21 bis 23, **dadurch gekennzeichnet, dass** zunächst Ibuprofen oder Ibuprofensalz mit hochdisperser Kieselsäure 1 h bis 5 h gemischt wird, dann die restlichen Zusatzstoffe untergemischt werden, diese Mischung walzenkompaktiert und anschließend durch ein Sieb gedrückt wird, so dass ein Granulat entsteht.

25. Verfahren zur Herstellung von Ibuprofenformulierungen einem der Ansprüche 21 bis 24, **dadurch gekennzeichnet, dass** zunächst Ibuprofen oder Ibuprofensalz mit hochdisperser Kieselsäure 1 h bis 5 h gemischt wird, dann die restlichen Zusatzstoffe untergemischt werden, diese Mischung walzenkompaktiert und anschließend durch ein Sieb gedrückt wird, so dass ein Granulat entsteht, und diesem Granulat mindestens 0,5 Gew.-% weitere hochdisperse Kieselsäure zugemischt werden.

26. Verfahren zur Herstellung von Ibuprofenformulierungen nach einem der Ansprüche 21 bis 25, **dadurch gekennzeichnet, dass** beim Mischprozess die Temperatur des Ibuprofens 50 °C nicht überschreitet.

27. Pharmazeutische Darreichungsform enthaltend eine Ibuprofenformulierung einem der Ansprüche 1 bis 26 hergestellt durch Verpressung.

28. Pharmazeutische Darreichungsform nach Anspruch 27, wobei der Ibuprofengehalt der Tablette mindestens 60 Gew.-% beträgt.

## Claims

1. A directly tabletable ibuprofen formulation comprising
a) 50-99% by weight of crystalline ibuprofen,
b) 1-15% by weight of a finely divided excipient with a surface area of at least 100 m²/g, and
c) 0-40% by weight of further excipients,
with the proviso that the total amount of components a) to c) corresponds to 100% by weight, comprising silica as finely divided excipient, and where at least 70% of the surface of the ibuprofen crystals are covered with the finely divided excipient.

2. The ibuprofen formulation according to claim 1, where the ibuprofen is present in the form of the free acid or as alkali metal or alkaline earth metal salt or as salt with a basic amine or amino acid.

3. The ibuprofen formulation according to claim 1 or 2, where the ibuprofen crystals have an average particle size of from 20 to 200 µm.

4. The ibuprofen formulation according to any of claims 1 to 3, wherein the finely divided excipient has a surface area of at least 150 m²/g.

5. The ibuprofen formulation according to any of claims 1 to 4, wherein the finely divided excipient has a surface area of at least 200 m²/g.

6. The ibuprofen formulation according to any of claims 1 to 5, wherein the finely divided excipient has a surface area of at least 250 m²/g.

7. The ibuprofen formulation according to any of claims 1 to 6, comprising hydrophobized silica as finely divided excipient.

8. The ibuprofen formulation according to any of claims 1 to 7, comprising
a) 50-94% by weight of ibuprofen,
b) 1-15% by weight of finely divided silica with a surface area of at least 100 m²/g,
c) 5-20% by weight of microcrystalline cellulose,
d) 0-10% by weight of crosslinked polyvinylpyrrolidone, crosslinked sodium carboxymethylcellulose or crosslinked sodium carboxymethyl starch,
e) 0-10% by weight of hydroxyalkylcellulose, and
f) 0-5% by weight of a conventional lubricant.

9. The ibuprofen formulation according to any of claims 1 to 8, comprising
a) 50-93.5% by weight of ibuprofen,
b) 1.5-10% by weight of finely divided silica with a surface area of at least 150 m²/g,
c) 5-15% by weight of microcrystalline cellulose,
d) 0-10% by weight of crosslinked polyvinylpyrrolidone, crosslinked sodium carboxymethylcellulose or crosslinked sodium carboxymethyl starch,
e) 0-10% by weight of hydroxyalkylcellulose, and
f) 0-5% by weight of a conventional lubricant.

10. The ibuprofen formulation according to any of claims 1 to 9, wherein fillers, binders, disintegrants and lubricants or mixtures thereof are employed as further conventional excipients.

11. The ibuprofen formulation according to any of claims 1 to 10, wherein microcrystalline cellulose, cellulose, calcium hydrogen phosphate, mannitol, sorbitol, xylitol or lactose are employed as fillers.

12. The ibuprofen formulation according to any of claims 1 to 11, wherein polyvinylpyrrolidone, vinylpyrrolidone/vinyl acetate copolymers, hydroxypropylmethylcellulose, hydroxypropylcellulose, hydroxyethylcellulose or methylcellulose are employed as binders.

13. The ibuprofen formulation according to any of claims 1 to 12, wherein crosslinked sodium carboxymethyl starch, crosslinked sodium carboxymethylcellulose or crosslinked polyvinylpyrrolidone are employed as disintegrants.

14. The ibuprofen formulation according to any of claims 1 to 13, wherein stearic acid, magnesium stearate, sodium stearyl fumarate, leucine, sodium benzoate or poloxamers are employed as disintegrants.

15. The ibuprofen formulation according to any of claims 1 to 14, where the ibuprofen crystals covered with the finely divided excipient are in agglomerated form with an average particle size of the agglomerates of between 150 and 1500 µm.

16. The ibuprofen formulation according to any of claims 1 to 15, where the agglomerates are in a form with an average particle size of between 200 and 1200 µm.

17. The ibuprofen formulation according to any of claims 1 to 16, where the agglomerates are in a form with an average particle size of between 250 and 1000 µm.

18. The ibuprofen formulation according any of claims 1 to 17, where the agglomerates are in a form with an average particle size of between 250 and 1000 µm, and the proportion of agglomerates with particle sizes of < 85 µm is less than 10% by weight.

19. The ibuprofen formulation according any of claims 1 to 18, where the agglomerates are in a form with an average particle size of between 250 and 1000 µm, and the proportion of agglomerates with particle sizes of < 85 µm is less than 6% by weight.

20. The ibuprofen formulation according to any of claims 1 to 19, where the ibuprofen crystals covered with the finely divided excipient are present in agglomerated form, and the surface of the agglomerates is at least 70% covered with the finely divided excipient.

21. A process for producing ibuprofen formulations according to any of claims 1 to 20 by mixing ibuprofen with a finely divided excipient, which comprises mixing ibuprofen with a finely divided excipient which has a surface area of at least 100 m²/g until at least 70% of the surface of the ibuprofen particles are covered with the finely divided excipient.

22. The process according to claim 21, wherein ibuprofen and finely divided excipient are mixed with a mixing time of 1 h to 5 h.

23. The process according to claim 21 or 22, wherein initially ibuprofen or ibuprofen salt is mixed with finely divided silica for 1h to 5 h, and then the remaining additives are mixed in.

24. The process for producing ibuprofen formulations according to any of claims 21 to 23, wherein initially ibuprofen or ibuprofen salt is mixed with finely divided silica for 1h to 5 h, then the remaining additives are mixed in, this mixture is roll-compacted and subsequently forced through a sieve to result in granules.

25. The process for producing ibuprofen formulations according to any of claims 21 to 24, wherein initially ibuprofen or ibuprofen salt is mixed with finely divided silica for 1 h to 5 h, then the remaining additives are mixed in, this mixture is roll-compacted and subsequently forced through a sieve to result in granules, and at least 0.5% by weight of further finely divided silica is admixed with these granules.

26. The process for producing ibuprofen formulations according to any of claims 21 to 25, wherein the temperature of the ibuprofen during the mixing process does not exceed 50°C.

27. A pharmaceutical dosage form comprising an ibuprofen formulation according to any of claims 1 to 26 produced by compression.

28. The pharmaceutical dosage form according to claim 27, where the ibuprofen content of the tablet is at least 60% by weight.

## Revendications

1. Formulation d'ibuprofène pouvant être directement mise sous forme de comprimés, contenant :
a) 50 à 99 % en poids d'ibuprofène cristallin,
b) 1 à 15 % en poids d'un adjuvant hautement dispersé ayant une surface d'au moins 100 m²/g et
c) 0 à 40 % en poids d'autres adjuvants,
à condition que la quantité totale des composants a) à c) soit de 100 % en poids, de la silice étant contenue en tant qu'adjuvant hautement dispersé et au moins 70 % de la surface des cristaux d'ibuprofène étant recouverte avec l'adjuvant hautement dispersé.

2. Formulation d'ibuprofène selon la revendication 1, dans laquelle l'ibuprofène se présente sous la forme de l'acide libre ou sous la forme d'un sel alcalin ou alcalino-terreux ou sous la forme d'un sel avec une amine basique ou un acide aminé.

3. Formulation d'ibuprofène selon la revendication 1 ou 2, dans laquelle les cristaux d'ibuprofène présentent une taille de particule moyenne de 20 à 200 µm.

4. Formulation d'ibuprofène selon l'une quelconque des revendications 1 à 3, **caractérisée en ce que** l'adjuvant hautement dispersé présente une surface d'au moins 150 m²/g.

5. Formulation d'ibuprofène selon l'une quelconque des revendications 1 à 4, **caractérisée en ce que** l'adjuvant hautement dispersé présente une surface d'au moins 200 m²/g.

6. Formulation d'ibuprofène selon l'une quelconque des revendications 1 à 5, **caractérisée en ce que** l'adjuvant hautement dispersé présente une surface d'au moins 250 m²/g.

7. Formulation d'ibuprofène selon l'une quelconque des revendications 1 à 6, contenant en tant qu'adjuvant hautement dispersé de la silice hydrophobée.

8. Formulation d'ibuprofène selon l'une quelconque des revendications 1 à 7, contenant :
a) 50 à 94 % en poids d'ibuprofène,
b) 1 à 15 % en poids de silice hautement dispersée ayant une surface d'au moins 100 m²/g,
c) 5 à 20 % en poids de cellulose microcristalline,
d) 0 à 10 % en poids de polyvinylpyrrolidone réticulée, de carboxyméthylcellulose sodique réticulée ou de carboxyméthyl-amidon sodique réticulé,
e) 0 à 10 % en poids d'hydroxyalkylcellulose et
f) 0 à 5 % en poids d'un lubrifiant usuel.

9. Formulation d'ibuprofène selon l'une quelconque des revendications 1 à 8, contenant :
a) 50 à 93,5 % en poids d'ibuprofène,
b) 1,5 à 10 % en poids de silice hautement dispersée ayant une surface de plus de 150 m²/g,
c) 5 à 15 % en poids de cellulose microcristalline,
d) 0 à 10 % en poids de polyvinylpyrrolidone réticulée, de carboxyméthylcellulose sodique réticulée ou de carboxyméthyl-amidon sodique réticulé,
e) 0 à 10 % en poids d'hydroxyalkylcellulose et
f) 0 à 5 % en poids d'un lubrifiant usuel.

10. Formulation d'ibuprofène selon l'une quelconque des revendications 1 à 9, **caractérisée en ce que** des charges, des liants, des désintégrants et des lubrifiants ou leurs mélanges sont utilisés en tant qu'adjuvants usuels supplémentaires.

11. Formulation d'ibuprofène selon l'une quelconque des revendications 1 à 10, **caractérisée en ce que** de la cellulose microcristalline, de la cellulose, de l'hydrogénophosphate de calcium, du mannitol, du sorbitol, du xylitol ou du lactose sont utilisés en tant que charges.

12. Formulation d'ibuprofène selon l'une quelconque des revendications 1 à 11, **caractérisée en ce que** de la polyvinylpyrrolidone, des copolymères de vinylpyrrolidone-acétate de vinyle, de l'hydroxypropylméthylcellulose, de l'hydroxypropylcellulose, de l'hydroxyéthylcellulose ou de la méthylcellulose sont utilisés en tant que liant.

13. Formulation d'ibuprofène selon l'une quelconque des revendications 1 à 12, **caractérisée en ce que** du carboxyméthyl-amidon sodique réticulé, de la carboxyméthylcellulose sodique réticulée ou de la polyvinylpyrrolidone réticulée sont utilisés en tant que désintégrant.

14. Formulation d'ibuprofène selon l'une quelconque des revendications 1 à 13, **caractérisée en ce que** de l'acide stéarique, du stéarate de magnésium, du fumarate de stéaryle sodique, de la leucine, du benzoate de sodium ou des poloxamères sont utilisés en tant que lubrifiant.

15. Formulation d'ibuprofène selon l'une quelconque des revendications 1 à 14, dans laquelle les cristaux d'ibuprofène recouverts avec l'adjuvant hautement dispersé se présentent sous forme agglomérée avec une talle de particule moyenne des agglomérats comprise entre 150 et 1 500 µm.

16. Formulation d'ibuprofène selon l'une quelconque des revendications 1 à 15, dans laquelle les agglomérats se présentent avec une taille de particule moyenne comprise entre 200 et 1 200 µm.

17. Formulation d'ibuprofène selon l'une quelconque des revendications 1 à 16, dans laquelle les agglomérats se présentent avec une taille de particule moyenne comprise entre 250 et 1 000 µm.

18. Formulation d'ibuprofène selon l'une quelconque des revendications 1 à 17, dans laquelle les agglomérats se présentent avec une taille de particule moyenne comprise entre 250 et 1 000 µm, et la proportion d'agglomérats ayant des tailles de particules < 85 µm est inférieure à 10 % en poids.

19. Formulation d'ibuprofène selon l'une quelconque des revendications 1 à 18, dans laquelle les agglomérats se présentent avec une taille de particule moyenne comprise entre 250 et 1 000 µm, et la proportion d'agglomérats < 85 µm est inférieure à 6 % en poids.

20. Formulation d'ibuprofène selon l'une quelconque des revendications 1 à 19, dans laquelle les cristaux d'ibuprofène recouverts avec l'adjuvant hautement dispersé se présentent sous forme agglomérée et la surface des agglomérats est recouverte à au moins 70 % avec l'adjuvant hautement dispersé.

21. Procédé de fabrication de formulations d'ibuprofène selon l'une quelconque des revendications 1 à 20, par mélange d'ibuprofène avec un adjuvant hautement dispersé, **caractérisé en ce que** l'ibuprofène est mélangé avec un adjuvant hautement dispersé qui présente une surface d'au moins 100 m²/g, jusqu'à ce qu'au moins 70 % de la surface des particules d'ibuprofène soit recouverte avec l'adjuvant hautement dispersé.

22. Procédé selon la revendication 21, **caractérisé en ce que** l'ibuprofène et l'adjuvant hautement dispersé sont mélangés pendant un temps de mélange de 1 h à 5 h.

23. Procédé selon la revendication 21 ou 22, **caractérisé en ce que** de l'ibuprofène ou un sel d'ibuprofène est tout d'abord mélangé avec de la silice hautement dispersée pendant 1 h à 5 h, puis les additifs restants sont mélangés.

24. Procédé de fabrication de formulations d'ibuprofène selon l'une quelconque des revendications 21 à 23, **caractérisé en ce que** de l'ibuprofène ou un sel d'ibuprofène est tout d'abord mélangé avec de la silice hautement dispersée pendant 1 h à 5 h, puis les additifs restants sont mélangés, ce mélange est compacté au cylindre, puis pressé au travers d'un tamis de manière à former un granulat.

25. Procédé de fabrication de formulations d'ibuprofène selon l'une quelconque des revendications 21 à 24, **caractérisé en ce que** de l'ibuprofène ou un sel d'ibuprofène est tout d'abord mélangé avec de la silice hautement dispersée pendant 1 h à 5 h, puis les additifs restants sont mélangés, ce mélange est compacté au cylindre, puis pressé au travers d'un tamis de manière à former un granulat, et ce granulat est mélangé avec au moins 0,5 % en poids de silice hautement dispersée supplémentaire.

26. Procédé de fabrication de formulations d'ibuprofène selon l'une quelconque des revendications 21 à 25, **caractérisé en ce que** la température de l'ibuprofène ne dépasse pas 50 °C pendant le procédé de mélange.

27. Forme pharmaceutique contenant une formulation d'ibuprofène selon l'une quelconque des revendications 1 à 26, fabriquée par compression.

28. Forme pharmaceutique selon la revendication 27, dans laquelle la teneur en ibuprofène des comprimés est d'au moins 60 % en poids.
